# EUROPÄISCHE PATENTANMELDUNG

(11) **EP 2 305 270 A1**
(43) Veröffentlichungstag der Anmeldung: **06.04.2011**
(21) Anmeldenummer: 10183673.2
(22) Anmeldetag: 16.02.2001
(51) Int. Cl.: A61K 31/7012, A61K 31/702, A61K 31/715, A61P 31/00

(54) **Antiadhäsive Kohlenhydrate**

(30) Priorität: 16.02.2000 DE 10006989
(62) Teilanmeldung aus: 01919291.3
(71) Anmelder: N.V. Nutricia, 2712 HM Zoetermeer (NL)
(72) Erfinder: Stahl, Bernd, DE-61191, Rosbach (DE); Boehm, Günther, DE-04107 Leipzig (DE)
(74) Vertreter: Swinkels, Bart Willem

(57) **Zusammenfassung**

Es wird ein pharmazeutisches oder diätetisches Präparat bereitgestellt, das zur Verringerung und/oder Blockierung der Adhäsion von pathogenen Substanzen und Organismen an eukaryontische Zellen, insbesondere Säugerzellen dient. Dieses Präparat enthält mindestens ein Kohlenhydrat mit einer Uronsäureeinheit an einem seiner Enden. 10 bis 100 % der vorhandenen, endständigen Uronsäureeinheiten der Kohlenhydrate besitzen dabei eine Doppelbindung, die insbesondere zwischen dem C₄- und C₅-Atom liegt.

## Beschreibung

Die Erfindung betrifft ein pharmazeutisches oder diätetisches Präparat zur Verringerung und/oder Blockierung der Adhäsion von pathogenen Substanzen und Organismen an eukaryontische Zellen, insbesondere Säugerzellen, das mindestens ein antiadhäsives Kohlenhydrat mit einer Uronsäureeinheit an einem seiner Enden enthält, und die Verwendung dieses Präparates und der darin enthaltenen Kohlenhydrate zu den genannten Zwecken.

Die Adhäsion sowohl pathogener Organismen als auch zellschädigender Substanzen an die Oberfläche der Säugerzellen ist der erste Schritt und eine unabdingbare Voraussetzung für eine Infektion bzw. Schädigung der Zelle. Die Interaktion zwischen den Pathogenen und den Zellen kommt durch eine Ligand-Rezeptor-Beziehung zustande, die damit ein wichtiger Virulenz- bzw. Toxizitätsfaktor der Pathogene ist. Unter Pathogene sind dabei zumindest Bakterien, Viren, Pilze, einzellige und mehrzellige Parasiten, Toxine und Schwermetallkationen zu verstehen. Bei dieser Ligand-Rezeptor-Beziehung spielen Glycostrukturen eine wichtige Rolle.

Eine Möglichkeit, diese Ligand-Rezeptor-Beziehung zumindest zu vermindern oder vollständig zu verhindern, besteht in der Blockierung der jeweiligen Rezeptoren auf der Zelloberfläche oder am Liganden.

Anhand spezifischer Testsysteme konnte gezeigt werden, daß verschiedene Kohlenhydratmischungen die Adhäsion von beispielsweise Mikroorganismen an die Zelloberfläche vermindern oder sogar vollständig verhindern, man vergleiche: Kunz, C.; Rudloff, S. Acta Paediatr. 1993, 82, 903-912. Es wird dabei angenommen, daß die wirksamen Kohlenhydrate eine weitgehende Analogie zu den Rezeptor- oder Ligandstrukturen besitzen. Bei den beschriebenen Untersuchungen wurden zahlreiche Kohlenhydrate sowohl tierischer als auch pflanzlicher Herkunft und auch Hydrolyseprodukte pflanzlicher Polysaccharide eingesetzt.

Die Zusammensetzung und Struktur der in der Natur vorkommenden Kohlenhydrate und beispielsweise der Kohlenhydrate der Humanmilch sind sehr komplex. Gleiches gilt aber auch für die Kohlenhydrate pflanzlicher Herkunft bzw. Hydrolyseprodukte von pflanzlichen Kohlenhydraten. Daraus ergibt sich, daß die festgestellte antiadhäsive Wirkung von Kohlenhydraten für Pathogene an Säugerzellen meist mit Kohlenhydratgemischen und nicht mit gereinigten Einzelstrukturen durchgeführt wurden.

So ist beispielsweise bekannt, daß wässrige Auszüge sowie Säfte aus verschiedenen Pflanzenprodukten gegen durch pathogene Keime hervorgerufene Erkrankungen im Intestinal- und Urogenitaltrakt wirksam sind. In der PCT/EP 94/03006 (WO 95107084) wird nun beschrieben, daß durch eine auf bestimmte Weise zubereitete Karottensuppe, Blasentee, Kokosmilch usw. die Adhärenz pathogener Keime an Epitelzellen des Gastrointestinal- und Urogenitaltraktes wesentlich reduziert wird. Für diese Wirkung sollen angeblich die in den Pflanzenprodukten vorhandenen Pektine verantwortlich sein, bei denen es sich im wesentlichen aus Ketten von 1,4-α-glycosidisch verbundenen Galakturoniden handelt. Die eigentlich wirksamen Galakturonide sollen dabei verschiedene Kriterien erfüllen, nämlich einen bestimmten Polymerisations- und Methylierungsgrad.

Aufgabe der vorliegenden Erfindung ist es, einen Weg aufzuzeigen, wie mit Hilfe von Kohlenhydraten die Adhäsion von Pathogenen durch Interaktion mit Liganden und/oder Oberflächenstrukturen von eukaryontischen Zellen und insbesondere Säugerzellen wirksamer verringert oder verhindert wird.

Gelöst wird diese Aufgabe durch die Lehre der Ansprüche.

Gegenstand der Erfindung ist somit unter anderem ein pharmazeutisches oder diätetisches Präparat, das mindestens ein antiadhäsives Kohlenhydrat mit einer Uronsäureeinheit an einem seiner Enden aufweist. Bekanntlich besitzen Kohlenhydrate zumindest zwei Enden und können auch drei oder mehr Enden besitzen, wenn sie verzweigt sind. Erfindungsgemäß können somit geradkettige Kohlenhydrate und auch verzweigtkettige Kohlenhydrate zum Einsatz gelangen. An einem dieser Enden befindet sich die genannte Uronsäureeinheit, die über eine endständige COOH-Gruppe verfügt, die verestert sein kann. Bevorzugte Uronsäuren bzw. Uronsäureeinheiten sind dabei folgende freie oder veresterte Säuren: Galacturonsäure, Glucuronsäure, Guluronsäure, Iduronsäure, Mannuronsäure, Riburonsäure und Altruronsäure, von denen die Galacturonsäure und die Glucuronsäure besonders bevorzugt sind.

Das erfindungsgemäße Präparat enthält mindestens ein antiadhäsives Kohlenhydrat und somit eine bestimmte Spezies mit einer Uronsäureeinheit an einem seiner Enden. Das erfindungsgemäße Präparat kann jedoch auch mehrere antiadhäsive Kohlenhydrate mit einer endständigen Uronsäureeinheit aufweisen. Zweckmäßigerweise enthält das erfindungsgemäße Präparat eine Mischung aus mehreren derartigen antiadhäsiven Kohlenhydraten.

Unter einem antiadhäsiven Kohlenhydrat wird im Rahmen der vorliegenden Unterlagen ein solches Kohlenhydrat verstanden, das über eine endständige Uronsäureeinheit verfügt, und zwar unabhängig davon, ob diese Uronsäureeinheit eine Doppelbindung aufweist oder nicht. Mit anderen Worten, der Begriff antiadhäsive Kohlenhydrate bezeichnet die Summe aus den Kohlenhydraten mit einer Uronsäureeinheit, die eine Doppelbindung aufweisen, und denjenigen Kohlenhydraten, die zwar über eine Uronsäureeinheit verfügen, die jedoch keine Doppelbindung besitzt. Ein wesentlicher Gedanke der Erfindung besteht nun darin, dass solche antiadhäsiven Kohlenhydrate eingesetzt werden, die einen Mindesgehalt an Uronsäureeinheiten mit einer Doppelbindung aufweisen.

Die antiadhäsiven Kohlenhydrate können einen bestimmten Polymerisationsgrad besitzen, der allgemein und somit auch hier als DP abgekürzt wird. Üblicherweise sind jedoch antiadhäsive Kohlenhydrate mit unterschiedlichen DP's vorhanden, wobei auch die antiadhäsiven Kohlenhydrate mit einem bestimmten Polymerisationsgrad bzw. DP unterschiedlich zusammengesetzt sein können. Mit anderen Worten, das erfindungsgemäße Präparat enthält mindestens eine bestimmte antiadhäsive Kohlenhydratspezies mit einer Uronsäureeinheit an einem seiner Enden. Diese Kohlenhydratspezies besitzt natürlich einen bestimmten DP. Ferner können mehrere unterschiedlich zusammengesetzte antiadhäsive Kohlenhydratspezies mit demselben DP vorhanden sein. Zudem können antiadhäsive Kohlenhydrate mit unterschiedlichem DP zugegen sein, wobei für jeden Polymerisationsgrad ein oder mehrere antiadhäsive Kohlenhydratspezies vorhanden sein können.

Mit der Definition der oben näher spezifizierten antiadhäsiven Kohlenhydrate, die an einem Ende eine Uronsäureeinheit aufweisen, ist über die Art der weiteren Saccharideinheiten oder Monomereinheiten, aus denen diese antiadhäsiven Kohlenhydrate zusammengesetzt sind, keine Aussage getroffen, es sei denn, daß das antiadhäsive Kohlenhydrat lediglich aus einer einzigen Einheit (Polymerisationsgrad = DP1), nämlich einer Uronsäureeinheit besteht. Sofern das antiadhäsive Kohlenhydrat einen DP1 besitzt, besteht es ausschließlich aus einer derartigen Uronsäureeinheit. Besitzt das antiadhäsive Kohlenhydrat einen DP2 oder höher, dann können die mit der Uronsäureeinheit verbundenen, weiteren Saccharideinheiten beliebiger Art sein.

Von den vorhandenen Üronsäureeinheiten der antiadhäsiven Kohlenhydrate müssen nun 10 bis 100 % eine Doppelbindung besitzen. Die %-Angabe gibt dabei die Anzahl der insgesamt vorhandenen Uronsäureeinheiten mit einer Doppelbindung an einem der Enden der antiadhäsiven Kohlenhydrate mit einer derartigen Uronsäureeinheit an, bezogen auf die Summe aus diesen Uronsäureeinheiten mit einer Doppelbindung und den ggf. vorhandenen Uronsäureeinheiten ohne eine derartige Doppelbindung an einem Ende des antiadhäsiven Kohlenhydrates bzw. der antiadhäsiven Kohlenhydrate.

Eine Uronsäureeinheit an einem Ende eines antiadhäsiven Kohlenhydrates wird im übrigen hier auch als endständige Uronsäureeinheit bezeichnet.

Die erfindungsgemäß zum Einsatz gebrachten antiadhäsiven Kohlenhydrate, die an einem Ende eine derartige Uronsäureeinheit aufweisen, können nun an dem anderen Ende (im Falle einer nicht verzweigten Kette), eine nicht-reduzierende Saccharideinheit oder auch eine reduzierende Saccharideinheit aufweisen. Vorzugsweise weisen 10 bis 100 % der eine endständige Uronsäureeinheit aufweisenden, antiadhäsiven Kohlenhydrate am anderen Ende (bei einer geraden Kette) oder an einem der anderen Enden (im Falle einer verzweigten Kette) eine derartige reduzierende Saccharideinheit auf. Mit anderen Worten, 10 bis 100 % der endständigen Uronsäureeinheiten befinden sich an einem nicht-reduzierenden Ende. Somit können sogar alle vorhandenen endständigen Uronsäureeinheiten am nicht-reduzierenden Ende vorhanden sein.

Vorzugsweise liegen 50 bis 100 % der Doppelbindungen zwischen dem C₄- und C₅-Atom der endständigen Uronsäureeinheiten. Auch in diesem Falle bezieht sich die %-Angabe auf die Anzahl der Doppelbindungen, unabhängig von dem DP der antiadhäsiven Kohlenhydrate und der diese antiadhäsiven Kohlenhydrate ausmachenden Saccharideinheiten. Die Detektion der Doppelbindung und somit der endständigen Uronsäureeinheiten mit einer derartigen Doppelbindung kann dabei spektroskopisch bei 235 nm unter Verwendung des molaren Extinktionskoeffizienten von 5500 I/mol cm erfolgen, man vergleiche TP Kravtchenko, 1. Arnould, AGJ Voragen & W. Polnik Carbohydr. Polymer 1992, 19, 237-242.

Die Bestimmung der Kohlenhydrate mit einem reduzierenden Ende erfolgt mittels der lodometrie entsprechend den Angaben in: Analytical Chemistry of Carbohydrates H. Scherz, G. Bonn, Herausgeber Thieme Organic Chemistry Monograph Series, Stuttgart, New York, Thieme Verlag 1998, Seite 32. Kohlenhydrate mit ausschließlich nicht-reduzierenden Enden können mit üblichen Analyseverfahren, wie Osmometrie Massenspektrometrie (z. B. MALDI-MS, ESI-MS) Chromatographie (z. B. GPC, HPAEC, HPLC) und Kapillarelektrophorese, oder durch eine Kombination dieser Verfahren bestimmt werden.

Die erfindungsgemäß zum Einsatz gebrachten, antiadhäsiven Kohlenhydrate können neben einer endständigen Uronsäureeinheit auch ein nicht-reduzierendes Ende aufweisen, indem beispielsweise ein reduzierendes Ende nachträglich in ein nicht-reduzierendes Ende überführt wurde. Dies kann beispielsweise durch eine Oxydation, Reduktion oder auch durch eine Verknüpfung des reduzierenden Endes mit anderen Molekülen erreicht werden. Zu diesen anderen Molekülen gehören beispielsweise Proteine, Lipide und technische Polymere, wobei (Neo)glycokonjugate erhalten werden. Diese nachträgliche Änderung des reduzierenden Endes hat keinen Einfluss auf die antiadhäsive Wirkung der erfindungsgemäßen zum Einsatz gebrachten, antiadhäsiven Kohlenhydrate. Diese antiadhäsiven Kohlenhydrate können somit auch an bekannten Trägern über ein "ehemals" reduzierendes Ende immobilisiert sein, beispielsweise an einem üblichen Träger.

Wenn somit 10 bis 100 % der vorhandenen endständigen Uronsäureeinheiten eine Doppelbindung besitzen, dann bedeutet dies natürlich auch, dass 0 bis 90 % der vorhandenen, endständigen Uronsäureeinheiten keine derartige Doppelbindung besitzen. Vorzugsweise besitzen 10 bis 50 % der vorhandenen, endständigen Uronsäureeinheiten des antiadhäsiven Kohlenhydrates oder der antiadhäsiven Kohlenhydrate eine Doppelbindung.

Es wurde nämlich überraschend gefunden, dass im Gegensatz zu der Lehre der eingangs genannten WO 95/07084 weder der Polymerisationsgrad noch der Methylierungsgrad für eine ausgeprägte antiadhäsive Funktion verantwortlich sind, auch wenn gegebenenfalls einige der dort beschriebenen Kohlenhydrate über eine derartige Funktion verfügen können. Eine ausgeprägte antiadhäsive Funktion üben vielmehr Kohlenhydrate mit einer eine Doppelbindung aufweisenden, endständigen Uronsäureeinheit auf. Derartige antiadhäsiven Kohlenhydrate und insbesondere solche, deren Uronsäureeinheit die Doppelbindung zwischen dem C₄- und C₅ werden jedoch nach der Lehre der genannten WO 95/07084 nicht erhalten, worauf nachstehend noch näher eingegangen wird.

Wenn erfindungsgemäß von einem antiadhäsiven Kohlenhydrat mit einem gegebenen Polymerisationsgrad die Rede ist, dann kann es sich dabei um nur ein einziges antiadhäsives Kohlenhydrat handeln. Es kann sich aber auch um mehrere unterschiedlich aufgebaute antiadhäsive Kohlenhydrate handeln, deren gemeinsame Merkmale einerseits der gegebene Polymerisationsgrad und andererseits die endständige Uronsäureeinheit sind.

Vorzugsweise weist das erfindungsgemäße Präparat nicht nur ein oder mehrere antiadhäsive Kohlenhydrate mit einem gegebenen DP sondern mehrere antiadhäsive Kohlenhydrate unterschiedlichen Polymerisationsgrades auf. Die zur Anwendung gebrachten antiadhäsiven Kohlenhydrate besitzen dabei vorzugsweise einen Polymerisationsgrad von DP2 bis DP 40 und insbesondere von DP 2 bis DP 10 und von maximal DP100. Vorzugsweise kommen somit Gemische von antiadhäsiven Kohlenhydraten mit unterschiedlicher Kettenlänge zum Einsatz. Auch in diesem Fall kann es sich bei einem antiadhäsiven Kohlenhydrat mit einer bestimmten Kettenlänge bzw. einem gegebenen Polymerisationsgrad um nur eine einziges Kohlenhydratspezies oder um mehrere oder eine beliebig große Vielzahl von antiadhäsiven Kohlenhydratspezies handeln.

Die antiadhäsiven Kohlenhydrate mit einer endständigen, insbesondere am nicht-reduzierenden Ende befindlichen, eine Doppelbindung aufweisenden Uronsäureeinheit verfügen somit über eine verstärkte antiadhäsive Wirkung. Diese antiadhäsiven Kohlenhydrate werden im Rahmen der vorliegenden Unterlagen auch als ungesättigte Kohlenhydrate bezeichnet.

Die erfindungsgemäß eingesetzten antiadhäsiven Kohlenhydrate und somit auch die ungesättigten antiadhäsiven Kohlenhydrate sind beispielsweise dadurch erhältlich, dass saure Kohlenhydrate und bevorzugt uronsäurehaltige Kohlenhydrate mittels Enzymen oder chemischer Spaltung derart gespalten werden, dass die angegebenen Gehalte an Uronsäureeinheiten mit einer Doppelbindung erhalten werden. Als bevorzugte Ausgangs-Kohlenhydrate können dabei folgende eingesetzt werden: Pektine, Pektate, Alginate, Chondroitine, Hyaluronsäuren, Heparine, Heparane, bakterielle Kohlenhydrate und andere uronsäurehaltige Kohlenhydrate. Bevorzugte Rohstoffe sind dabei Pflanzen und/oder Pflanzenteile (wie Karotten, Zitrusfrüchte, Rüben und Äpfel, man vergleiche C. Rolin, BU Nielsen, & PE Glahn in Polysaccarides (ed. S. Dimitriu), Marcel Dekker New York 1998. Auch können Algen, tierische Gewebe und bakterielle Produkte Anwendung finden.

Werden die ungesättigten antiadhäsiven Kohlenhydrate durch chemische Spaltung hergestellt, dann ist diese derart durchzuführen, dass eine Doppelbindung über eine β-Elimination eingeführt wird, indem beispielsweise Pektine unter neutralen oder schwach basischen Bedingungen gespalten werden, man vergleiche MJH Keijbets & W. Pilnik Carbohydr. Res. 1974, 33, 359-362.

Die enzymatische Spaltung wird insbesondere mit Hilfe von Lyasen (wie Pektinlyasen oder Pektatlyasen) oder Lyase-haltigen Enzympräparationen durchgeführt.

Im Falle der chemischen Spaltung arbeitet man bei neutralen bis alkalischen Bedingungen, um dadurch den gewünschten Gehalt an Doppelbindungen zu erhalten. Durch die geeignete Wahl der weiteren Parameter wie Temperatur, pH und Pufferkonzentration kann auch der Veresterungsgrad der Carboxylgruppe und/oder Hydroxylgruppe beeinflusst werden. Bei höheren Veresterungsgraden der eingesetzten Ausgangsverbindungen (z. B. Pektine) können die erfindungsgemäß eingesetzten antiadhäsiven Kohlenhydrate und somit auch die ungesättigten antiadhäsiven Kohlenhydrate ebenfalls bei einer im schwach sauren Bereich durchgeführten Spaltung erhalten werden.

Die antiadhäsive Wirkung der ungesättigten antiadhäsiven Kohlenhydrate wird auch durch das Vorhandensein von Methylestern der Carboxylgruppe als auch von Acetylestern z.B. am C-2 und/oder C-3 Atom der Uronsäuren beeinflusst. Dies gilt insbesondere bei den Galacturonsäuren der Pektine. Der Grad der Methylierung bzw. Acetylierung beträgt vorzugsweise 20 bis 75 % und insbesondere 20 bis 50%.

Wie bereits dargelegt, kommt es bei dem erfindungsgemäß vorzugsweise zum Einsatz gebrachten Gemisch aus antiadhäsiven Kohlenhydraten insbesondere auf die Doppelbindung der insbesondere am nicht-reduzierenden Ende befindlichen Uronsäureeinheiten an. Bei den weiteren, mit dieser eine Doppelbindung oder auch keine derartige Doppelbindung aufweisenden Uronsäureeinheit verbundenen Saccharideinheiten kann es sich um ausschließlich saure Kohlenhydrateinheiten, um ausschließlich neutrale Kohlenhydrateinheiten und um eine Mischung aus sauren und neutralen Kohlenhydrateinheiten handeln. So beeinflussen nämlich auch die neutralen Kohlenhydrateinheiten die antiadhäsive Wirkung der ungesättigten antiadhäsiven Kohlenhydrate. Es handelt sich dabei im wesentlichen um Rhamnose, Arabinose, galaktose, Xylose, Glucose, Fucose und Apiose, die ihrerseits wieder mit Feroylresten und phenolischen Substanzen verknüpft sein können. Dies gilt insbesondere für Pektine. Der Anteil neutraler Kohlenhydrateinheiten beträgt dabei vorzugsweise maximal 50 % und insbesondere 0 bis 30 %.

Die antiadhäsive Wirkung des zum Einsatz gebrachten antiadhäsiven Kohlenhydrates oder des Gemisches von antiadhäsiven Kohlenhydraten ist nicht von der Konzentration in einem Endprodukt, sondern von der Zufuhrmenge abhängig. Somit kann das erfindungsgemäße Präparat ausschließlich aus einem antiadhäsiven Kohlenhydrat oder aus einem Gemisch von antiadhäsiven Kohlenhydraten bestehen. Dazu wird das Präparat beispielsweise als Tablette oder als Nahrungsmittelsupplement formuliert. Selbstverständlich können im Falle eines pharmazeutischen Präparates übliche, pharmakologisch verträgliche Träger, Verdünnungsmittel und/oder Hilfsstoffe vorhanden sein. Auch können diese antiadhäsiven Kohlenhydrate einem beliebigen Nahrungsmittel oder pharmazeutischem Präparat einverleibt werden, die weitere Ingredienzien enthalten. Im Falle von Nahrungsmitteln kann es sich dabei um Fette, Proteine, Mineralien, Spurenelemente, Vitamine und andere zur Herstellung von Nahrungen geeignete Materialien handeln. Zudem ist es möglich, die erfindungsgemäß zum Einsatz gebrachten antiadhäsiven Kohlenhydrate mit anderen Kohlenhydraten beliebiger Art zusammen zum Einsatz zu bringen.

Nach einer bevorzugten Ausführungsform handelt es sich bei den anderen Kohlenhydraten um eine präbiotische Kohlenhydratmischung gemäß der Lehre der WO 00/08948 mit dem internationalen Aktenzeichen PCT/EP99/05878 und somit um eine präbiotische Kohlenhydratmischung aus zwei unterschiedlichen, im wesentlichen löslichen Kohlenhydratkomponenten A und B, die im Magen-Darm-Trakt unverdaut bleiben und nicht resorbiert bis zum Dickdarm gelangen, enthält, wobei die Kohlenhydratkomponente A aus mindestens einem Monosaccharid oder aus mindestens einem Oligosaccharid (Disaccharid bis zu Hexasaccharid) oder aus einer Mischung aus zweien oder mehreren dieser Saccharide aufgebaut ist, wobei die Kohlenhydratkomponente B aus einem Polysaccharid (ab Heptasaccharid) oder aus einer Mischung aus zwei oder mehreren Polysacchariden aufgebaut ist, wobei die Kohlenhydratkomponente A = 5 bis 95 Gew.-% und die Kohlenhydratkomponente B = 5 bis 95 Gew.-% der Summe der Kohlenhydratkomponenten A + B ( = 100 Gew.-%) ausmachen, und wobei mindestens 80 Gew.-% der Kohlenhydrate/Saccharide der Kohlenhydratkomponente A und B präbiotisch wirken. Für die Zwecke der vorliegenden Erfindung können allerdings nur solche Kohlenhydrate, die keine antiadhäsiven uronsäurehaltigen Kohlenhydrate darstellen, die Kohlenhydratkomponente A und die Kohlenhydratkomponente B ausmachen. Somit werden den Komponenten A und B keine antiadhäsiven Kohlenhydrate zugerechnet. Diese Kohlenhydrate, welche die Kohlenhydratkomponente A und die Kohlenhydratkomponente B ausmachen, werden nachstehend aus Gründen der einfacheren Darstellbarkeit als präbiotische Kohlenhydrate bezeichnet, obwohl nur ein Teil dieser Kohlenhydrate wirklich präbiotisch wirkt.

Mindestens 80 Gew.-% der als präbiotisch bezeichneten Kohlenhydrate bzw. Saccharide der Summe der Kohlenhydratkomponente A und B wirken somit präbiotisch. Vorzugsweise wirken mindestens 80 Gew.-% der zur Kohlenhydratkomponente A gehörigen, präbiotisch bezeichneten Kohlenhydrate und auch mindestens 80 Gew.-% der zur Kohlenhydratkomponente B gehörenden päbiotisch. Anders ausgedrückt, vorzugsweise mindestens jeweils 80 Gew.-% der präbiotisch bezeichneten Kohlenhydrate bzw. der Saccharide der Kohlenhydratkomponenten A und B müssen unverdaut (und daher nicht im Dünndarm resorbierbar) in den Dickdarm gelangen. Mit anderen Worten, diese Kohlenhydrate bzw. Saccharide der Kohlenhydratkomponenten A und B werden im Magen-Darm-Trakt weder im Magen noch im Dünndarm resorbiert und verdaut, sondern gelangen als solche in den Dickdarm.

Als lösliche Kohlenhydrate der Kohlenhydratkomponente A und B sind solche zu verstehen, die in Wasser in einer Konzentration von mindestens 1g/l bei Raumtemperatur eine homogene Lösung im physikalischen Sinne (z.B. gemäß Römpps Chemie Lexikon) bilden.

Der Anteil der nicht präbiotisch wirkenden Kohlenhydrate bzw. Saccharide bei den Kohlenhydratkomponenten A und B beträgt somit maximal 20 Gew.-%. Bei diesen Kohlenhydraten bzw. Sacchariden handelt es sich um solche, die zwar löslich sind, jedoch unverdaut ausgeschieden werden können. Diese Kohlenhydrate können einen physikalischen Effekt bewirken, indem Sie beispielsweise das Stuhlvolumen erhöhen oder aber eine Wasserbindung ausüben.

Vorzugsweise besitzen. die präbiotischen Kohlenhydrate/Saccharide, welche die Kohlenhydratkomponente A ausmachen, eine andere Struktur als die präbiotischen Kohlenhydrate/Saccharide, welche die Kohlenhydratkomponente B ausmachen. Weiterhin bevorzugt sind mindestens 80 Gew.-% der präbiotischen Kohlenhydrate/ Saccharide der Kohlenhydratkomponente A und B solche, welche Milchsäurebakterien fördern und/oder bifidogen sind. Der Gewichtsanteil der Kohlenhydratkomponente A ist dabei vorzugsweise größer ist als der Gewichtsanteil der Kohlenhydratkomponente B. Die Kohlenhydratkomponente A macht vorzugsweise 95 bis 60 Gew.-% und insbesondere ca. 90 Gew.-% aus während die Kohlenhydratkomponente B vorzugsweise 5 bis 40 Gew.-% und insbesondere ca. 10 Gew.-% ausmacht, wobei A + B = 100 Gew.-%. Die präbiotischen Kohlenhydrate/Saccharide der Kohlenhydratkomonenten A und B weisen insbesondere keine Glucoseeinheiten in α1-4 und/oder in α1-6-Bindung auf. Die präbiotischen Kohlenhydrate/Saccharide der Kohlenhydratkomponente B sind dabei vorzugsweise aus maximal bis zu 100 Monosaccharideinheiten aufgebaut.

Weiterhin bevorzugt gehören mindestens 60 Gew.-% und insbesondere 80 bis 100 Gew.-% der präbiotischen Kohlenhydrate/Saccharide der Kohlenhydratkomponente A zur Gruppe der Galactooligosaccharide und mindestens 60 Gew.-% und insbesondere 80 bis 100 Gew.-% der präbiotischen Kohlenhydrate/Saccharide der Kohlenhydratkomponente B zur Gruppe der Fructopolysaccharide.

Ist eine derartige präbiotische Kohlenhydratmischung in den erfindungsgemäßen Präparaten vorhanden, dann beträgt das Gewichtsverhältnis von dem oder den antiadhäsiven Kohlenhydrat(en) zu der präbiotischen Kohlenhydratmischung vorzugsweise 1 : 99 bis 99 : 1 und insbesondere 1 : 1.0 bis 10 : 1 und weiterhin insbesondere ca. 1 : 1.

Ferner können in den erfindungsgemäßen Präparaten neben den antiadhäsiven Kohlenhydraten und neben der gegebenenfalls vorhandenen präbiotischen Kohlenhydratmischung auch noch weitere übliche Kohlenhydrate beliebiger Art zugegen sein. Es kann sich dabei um unlösliche Kohlenhydrate, um lösliche sowie verdaubare Kohlenhydrate, um übliche primär einem nutritiven Zweck dienende Kohlenhydrate (z. B. Stärke, Maltodextrine, Lactose und Saccharose) oder um eine Mischung aus einem oder mehreren dieser Kohlenhydrate handeln. Die antiadhäsiven Kohlenhydrate machen in diesen Fällen vozugsweise 0,1 bis 30 und insbesondere 1 bis 10 Gew.-% aus.

Mit den antiadhäsiven Oligosacchariden wird erreicht, dass pathogene Substanzen nicht an Säugerzellen binden oder bereits gebundene Pathogene abgelöst werden. Durch den Zusatz von präbiotischen Oligosacchariden wird erreicht, dass die häufig im Zusammenhang mit Pathogenen auftretende Störung der Darmflora behoben wird. Darüberhinaus werden Pathogene an anderen Stellen außerhalb des gastointestinalen Traktes wie beispielsweise dem Urogenitaltrakt, dem respiratorischen Trakt, dem Blutsystem und der Haut durch die systemische Wirkung einer balancierten Darmflora bekämpft.

Da die Adhäsion von Pathogenen eine Voraussetzung für deren Infektiösität bzw. Toxizität an alle Zellen des Säugetierorganismus ist, können die erfindungsgemäß eingesetzten antiadhäsiven Kohlenhydrate nicht nur für die Verhinderung oder Verringerung von Infektionen bzw. Schädigungen im Magen-Darm-Trakt sondern bei allen Zellen eingesetzt werden.

Gegenstand der Erfindung ist somit auch die Verwendung der erfindungsgemäßen Präparate und der darin zum Einsatz gebrachten antiadhäsiven Kohlenhydrate zur Verhinderung oder Verringerung der Adhäsion von Pathogenen an eukaryontische Zellen und insbesondere Säugerzellen. Vorzugsweise werden diese Kohlenhydrate zur Behandlung von Infektionen des Gastrointestinaltraktes, des Blutsystems, der Atemwege, des Urogenitaltraktes, des Nasen-Rachenraumes und zur Behandlung von Schädigungen durch Toxine oder Schwermetallkationen der Zellen des Gastrointestinaltraktes, des Blutsystems, der Atemwege, des Urogenitaltraktes sowie des Nasen-Rachenraumes verwendet. Gegenstand der Erfindung ist somit auch die Verwendung der eingesetzten antiadhäsiven Kohlenhydrate zur Herstellung eines diätetischen oder pharmazeutischen Präparates für die genannten Behandlungszwecke.

Die Verwendung ist im übrigen nicht auf enteral applizierbare Nahrungsmittel oder pharmazeutische Präparate begrenzt. Vielmehr können die erfindungsgemäß eingesetzten antiadhäsiven Kohlenhydrate auch als Wirkstoff in nicht enteral applizierbaren pharmazeutischen Präparaten eingesetzt werden. Bei den erfindungsgemäßen Präparaten kann es sich somit auch um derartige nicht enteral applizierbare pharmazeutische Präparate handeln.

Die Zufuhrmenge der erfindungsgemäß eingesetzten antiadhäsiven Kohlenhydrate und somit aus der Summe aus Kohlenhydraten, die eine endständige Uronsäureeinheit ohne eine Doppelbindung aufweisen, und aus Kohlenhydraten, die ebenfalls eine endständige Uronsäureeinheit jedoch mit einer Doppelbindung aufweisen (ungesättigte Kohlenhydrate), wobei 10 bis 100 % der vorhandenen endständigen Uronsäureeinheiten eine derartige Doppelbindung aufweisen, beträgt mindesten 8 mg/kg Körpergewicht und Tag, vorzugsweise 8 bis 20 mg/kg Körpergewicht und Tag und insbesondere etwa 10 mg/kg und Tag. Diese Angabe bezieht sich insbesondere bevorzugt auf die ungesättigten antiadhäsiven Kohlenhydrate alleine.

Wenn im Rahmen der vorliegenden Unterlagen von Bereichen die Rede ist, seien es nun beispielsweise %-Bereiche oder mg-Bereiche, dann sind mit diesen Bereichsangabe alle Zwischenwerte und somit alle zwischen den Endwerten liegenden Werte und auch alle von den Bereichen umfassten engeren Bereiche offenbart und beansprucht. Die Angabe 8 bis 20 mg/kg umfasst somit alle dazischen liegende Werte und insbesondere ganzzahligen Wert, z.B. 9, 10, 11, 12, 13, 14, 15, 16, 17, 18, 19 mg/kg. Die Bereichsangabe 10 bis 100 % stellt somit lediglich ein verkürzte Angabe für alle denkbaren Zwischenwerte und insbesondere für die ganzzahligen Werte 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90, 91, 92, 93, 94, 95, 96, 97, 98 und 99 % dar. Dies gilt zum Beispiel für die %-Angaben für den Anteil an Uronsäureeinheiten mit einer Doppelbindung. Auch sämtliche denkbaren engeren Bereiche sind durch diese Angabe mit umfaßt und offenbart. sind. Analoges gilt für die Bereichsangaben, die sich auf Gew.-%, DP oder andere Einheiten beziehen.

In den nachstehenden Beispielen sind bevorzugte erfindungsgemäße Präparate beschrieben. Die Beispiele 1 bis 7 befassen sich mit der Herstellung von antiädhäsiven Kohlenhydraten, wobei mindestens 10 % der vorhanden Uronsäureeinheiten eine Doppelbindung aufweisen. Die dabei erhaltenen Produkte stellen Präparate dar, die ausschließlich aus antiadhäsiven Kohlenhydraten aufgebaut sind. Die Beispiele 8 und 13 beschreiben Mischungen aus antiadhäsiven Kohlenhydraten und einer präbiotischen Kohlenhydratmischung in verschiedenen Gewichtsverhältnissen

### Beispiel 1 (enzymatische Spaltung)

10 g GENU Pektin USP/100 (Fa. Hercules, Kopenhagen, DK) werden in 1 1 50 mM NaOAc Puffer (pH 5,0) gelöst. Zu dieser Lösung werden 10 ml Pectin-Lyase-Lösung (Sigma, Deisenhofen) gegeben. Die Umsetzung erfolgt bei 40 °C für 24 h. Die Reaktion wird durch Erhitzen auf 100 °C für 10 min gestoppt. Das Enzym und nicht umgesetztes Pektin werden über Filtration mit einer 50 kDa Membran entfernt. Das Filtrat wird anschließend gefriergetrocknet.

### Beispiel 2 (chemische Spaltung)

10 g GENU Pektin USP/100 (Fa. Hercules, Kopenhagen, DK) werden in 1 0,2 M NH₃-Carbonat Puffer (pH 6,8) gelöst und für 8 h bei 80 °C erhitzt. Die entstandenen Oligogalacturonide werden als Metallsalz (z. B. Bariumsalze) gefällt, filtriert, gewaschen, getrocknet, mittels Dowex-50 H⁺-Ionenaustauscher in die freie Säure überführt und gefriergetrocknet.

### Beispiel 3 (enzymatische Spaltung)

10 g Laboron Pektin X-77 A (C.C.A. Klimmeck, Bad Zwischenahn) werden in 1 1 50 mM Natriumacetat-Puffer (pH 4,5) gelöst. Der Verdau wird mit 1 ml Pectin-Lyase-Lösung (Fa. Gist-Brocades, Seclin, Frankreich) bei 45 °C für 24 h durchgeführt. Die Reaktion wird durch Erhitzen auf 95 °C für 5 min abgestoppt. Das Enzym und nicht umgesetztes Pektin werden über Gelfiltration mit BioGel P2 oder TosoHaas HW 40 S entfernt. Die Fraktion der Oligosaccharide wird anschließend gefriergetrocknet.

### Beispiel 4 (enzymatische Spaltung)

10 g Grünband-Pektin (Obipektin, Bischofszell, Schweiz) werden in 1 I. 50 mM Natriumacetat-Puffer (pH = 4,5) gelöst. Dazu werden 2 ml Pectinex 3 XL (Fa. Novo Nordisk, Dittingen, Schweiz) gegeben. Die Lösung wird 24 h auf 50 °C erhitzt. Das Abstoppen der Reaktion erfolgt durch Erhitzen auf 95 °C für 5 min. Die entstandenen Oligogalacturonide werden mit Ethanol gefällt, gewaschen und getrocknet.

### Beispiel 5 (chemische Spaltung)

10 g Grünband-Pektin (Obipektin, Bischofszell, Schweiz) werden in 1 I 0,1 M Natriumphosphat-Puffer (pH 6,8) gelöst und 1 h auf 90 °C erhitzt. Die freigesetzten Oligogalacturonide werden mit Ethanol gefällt, gewaschen und getrocknet.

### Beispiel 6 (chemische Spaltung)

10 g GENU Pektin USP/100 (Fa. Hercules, Kopenhagen, Dänemark) werden in 1 I 0,1 M Natriumphosphat-Puffer (pH 6,8) gelöst und 1 h auf 95 °C erhitzt. Langkettige Polymere werden mit Salzsäure bei pH 2 gefällt und abzentrifugiert. Der Überstand mit den Oligogalacturoniden wird lyophilisiert.

### Beispiel 7 (enzymatische Spaltung)

10 g Alginat werden 1 I 50 mM NaAc Puffer (pH 4,6) gelöst. Zu dieser Lösung werden 10 ml Alginat-Lyase-Lösung gegeben. Die Spaltung erfolgt bei 40 °C für 24 h. Die Reaktion wird durch Erhitzen auf 100 °C für 10 min gestoppt. Das Enzym und nicht umgesetztes Alginat werden über Filtration mit einer 50 kDa Membran entfernt. Das Filtrat wird anschließend gefriergetrocknet.

### Beispiele 8 bis 13

Zur Herstellung eines Präparates, das neben antiadhäsiven Kohlenhydraten auch präbiotische Kohlenhydrate enthält, wird wie folgt vorgegangen.

10 g Oligogalactoronide, die entweder hergestellt wurden durch enzymatische Spaltung gemäß einem der Beispiele 1, 3, 4 und/oder 7, oder die hergestellt wurden durch chemische Spaltung gemäß einem der Beispiele 2, 5 und/oder 6, werden vor dem Trocknen mit 10 g einer präbiotischen Kohlenhydrat-Mischung aus 9 Teilen Galacto-Oligosacchariden (z.B. Elixor Fa. Borculo und Oligomate Fa. Yakult) und 1 Teil hochmolekularem Inulin (z.B. Raftiline HP Fa. Orafti oder Frutafit TEX oder EXL. Fa Sensus oder Fibruline LC HAT Fa. Cosucra) gemäß den in der folgenden Tabelle aufgeführten Mengenanteilen vermengt und vermischt.

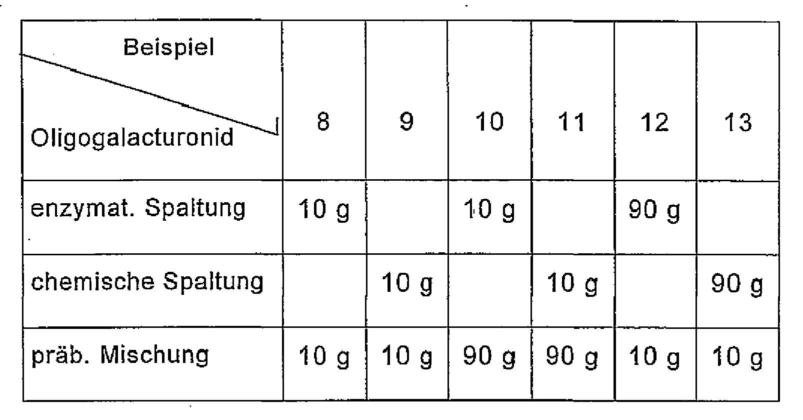

Statt der oben erwähnten präbiotischen Kohlenhydrat-Mischung aus Galacto-Oligosacchariden und Inulin können auch KohlenhydratMischungen eingesetzt werden, die aus folgenden Bestandteilen bestehen:
α-Galacto-Oligosacchariden und Inulin, β-Galacto-Oligosacchariden und Galactomannanen, Fructo-Oligosacchariden und Galactomannanen, Frucfto-Öligosacchariden und Arabinogalactanen, β-Galactooligosacchariden und Arabinogalactanen sowie Xylo-Oligosacchariden und Galactomannanen.

## Patentansprüche

1. Pharmazeutisches oder diätetisches Präparat, das ein antiadhäsives Kohlenhydrat oder mehrere antiadhäsive Kohlenhydrate mit einer Uronsäureeinheit an einem seiner bzw. ihrer Enden (endständige Uronsäureeinheit) enthält, **dadurch gekennzeichnet, dass** 10 bis 100 % der vorhandenen, endständigen Uronsäureeinheiten der antiadhäsiven Kohlenhydrate eine Doppelbindung besitzen.

2. Pharmazeutisches oder diätetisches Präparat nach Anspruch 1, **dadurch gekennzeichnet, dass** 10 bis 100 % der eine endständige Uronsäureeinheit aufweisenden, antiadhäsiven Kohlenhydrate ein reduzierendes Ende und an einem anderen Ende die endständige Uronsäureeinheit aufweisen.

3. Pharmazeutisches oder diätetisches Präparat nach Anspruch 1 oder 2, **dadurch gekennzeichnet, dass** 50 bis 100 % der Doppelbindungen zwischen dem C₄-und C₅-Atom der endständigen Uronsäureeinheiten liegen.

4. Pharmazeutisches oder diätetisches Präparat nach einem der vorhergehenden Ansprüche, **dadurch gekennzeichnet, dass** 10 bis 50 % der vorhandenen, endständigen Uronsäureeinheiten der antiadhäsiven Kohlenhydrate eine Doppelbindung besitzen.

5. Pharmazeutisches oder diätetisches Präparat nach einem der vorhergehenden Ansprüche, **dadurch gekennzeichnet, dass** es mehrere antiadhäsive Kohlenhydrate mit einer endständigen Uronsäureeinheit enthält, die einen unterschiedlichen Polymerisationsgrad besitzen.

6. Pharmazeutisches oder diätetisches Präparat nach einem der vorhergehenden Ansprüche, **dadurch gekennzeichnet, dass** die antiadhäsiven Kohlenhydrate einen maximalen Polymerisationsgrad von DP 100 und insbesondere von DP 2 bis DP 40 und weiterhin insbesondere DP 2 bis DP 10 besitzen.

7. Pharmazeutisches oder diätetisches Präparat nach einem der vorhergehenden Ansprüche, **dadurch gekennzeichnet, dass** die antiadhäsiven Kohlenhydrate mit einer endständigen Uronsäureeinheit ausgehend von uronsäurehaltigen Kohlenhydraten erhältlich sind, die derart chemisch oder enzymatisch gespalten werden, dass die angegebenen Gehalte an endständigen Uronsäureeinheiten mit einer Doppelbindung erhalten werden.

8. Pharmazeutisches oder diätetisches Präparat nach einem der vorhergehenden Ansprüche, **dadurch gekennzeichnet, dass** der Gehalt der in den vorhergehenden Ansprüchen beschriebenen antiadhäsiven Kohlenhydraten an neutralen Zuckereinheiten maximal 50 % und insbesondere 0 bis 30 % beträgt.

9. Pharmazeutisches oder diätetisches Präparat nach einem der vorhergehenden Ansprüche, **dadurch gekennzeichnet, dass** der Veresterungsgrad der in den vorhergehenden Ansprüchen beschriebenen antiadhäsiven Kohlenhydrate mit Methanol bis zu 75 % und insbesondere 20 bis 50 % beträgt.

10. Pharmazeutisches oder diätetisches Präparat nach einem der vorhergehenden Ansprüche, **dadurch gekennzeichnet, dass** dass es neben dem oder den antiadhäsiven Kohlenhydrat (en) eine präbiotische Kohlenhydratmischung aus zwei unterschiedlichen, im wesentlichen löslichen Kohlenhydratkomponenten A und B, die im Magen-Darm-Trakt unverdaut bleiben und nicht resorbiert bis zum Dickdarm gelangen, enthält, dass die Kohlenhydratkomponente A aus mindestens einem Monosaccharid oder aus mindestens einem Oligosaccharid (Disaccharid bis zu Hexasaccharid) oder aus einer Mischung aus zweien oder mehreren dieser Saccharide aufgebaut ist, die Kohlenhydratkomponente B aus einem Polysaccharid (ab Heptasaccharid) oder aus einer Mischung aus zwei oder mehreren Polysacchariden aufgebaut ist, dass die Kohlenhydratkomponente A = 5 bis 95 Gew.-% und die Kohlenhydratkomponente B = 5 bis 95 Gew.-% der Summe der Kohlenhydratkomponenten A + B (= 100 Gew.-%) ausmachen, und dass mindestens 80 Gew.-% der Kohlenhydrate/Saccharide der Kohlenhydratkomponente A und B präbiotisch wirken und dass die Kohlenhydrate, welche die Kohlenhydratkomponente A und die Kohlenhydratkomponente B ausmachen, keine antiadhäsiven Kohlenhydrate gemäss Anspruch 1 darstellen.

11. Pharmazeutisches oder diätetisches Präparat nach Anspruch 10, **dadurch gekennzeichnet, dass** das Gewichtsverhältnis von dem oder den antiadhäsiven Kohlenhydrat (en) zu der präbiotischen Kohlenhydratmischung 1 : 99 bis 99 : 1 und insbesondere 1 : 10 bis 10 : 1 und weiterhin insbesondere ca. 1 : 1 beträgt.

12. Diätetisches Präparat nach einem der Ansprüche 1 bis 11, **dadurch gekennzeichnet, dass** es übliche Nahrungsmittelinhaltsstoffe und Nahrungsmittelbestandteile einschliesslich Vitaminen und Spurenelementen enthält.

13. Verwendung der in einem der Ansprüche 1 bis 9 beschriebenen antiadhäsiven Kohlenhydrate mit einer endständigen Uronsäureeinheit zur Verringerung oder Verhinderung der Adhäsion von Pathogenen an eukaryontische Zellen.

14. Verwendung der antiadhäsiven Kohlenhydrate nach Anspruch 13 in einer Menge von mindestens 8 mg/kg und Tag und insbesondere 8 bis 20 mg/kg und Tag.

15. Verwendung nach Anspruch 13 oder 14 zur Behandlung von Infektionen des Gastrointestinaltraktes, des Blutsystemes, der Atemwege, des Urogenitaltraktes und des Nasen-Rachenraumes und zur Behandlung von durch Toxine oder Schwermetallkationen hervorgerufenen Schädigungen der Zellen des Gastrointestinaltraktes, des Blutsystemes, der Atemwege, des Urogenitaltraktes und des Nasen-Rachenraumes.
